(19) **Europäisches Patentamt / European Patent Office / Office européen des brevets**

(11) **EP 1 918 754 B1**

(12) **EUROPÄISCHE PATENTSCHRIFT**

(45) Veröffentlichungstag und Bekanntmachung des Hinweises auf die Patenterteilung:
**08.12.2010 Patentblatt 2010/49**

(51) Int Cl.:
*G02B 21/00* (2006.01)    *A61B 5/00* (2006.01)
*G01B 9/02* (2006.01)    *A61B 19/00* (2006.01)

(21) Anmeldenummer: **07019794.2**

(22) Anmeldetag: **10.10.2007**

(54) **Operationsmikroskop mit OCT-System**

Operation microscope with OCT system

Microscope d'opération doté d'un système OCT

(84) Benannte Vertragsstaaten:
**DE ES FR GB IT**

(30) Priorität: **06.11.2006 DE 102006052513**
**24.04.2007 DE 102007019679**

(43) Veröffentlichungstag der Anmeldung:
**07.05.2008 Patentblatt 2008/19**

(73) Patentinhaber: **Carl Zeiss Surgical GmbH**
**73447 Oberkochen (DE)**

(72) Erfinder:
• **Reimer, Peter**
**73479 Ellwangen (DE)**

• **Abele, Alfons**
**73527 Schwäbisch Gmünd (DE)**
• **Hauger, Christoph**
**73431 Aalen (DE)**
• **Seesselberg, Markus**
**73431 Aalen (DE)**

(74) Vertreter: **Gauss, Nikolai**
**c/o Carl Zeiss AG**
**Patentabteilung**
**Carl-Zeiss-Strasse 22**
**73447 Oberkochen (DE)**

(56) Entgegenhaltungen:
**EP-A- 0 815 801    EP-A- 1 220 004**
**EP-A- 1 231 496    DE-A1-102005 005 568**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft ein Operationsmikroskop mit einer Mikroskop-Abbildungsoptik, die ein Mikroskop-Hauptobjektivsystem sowie ein Vergrößerungssystem mit variabler Vergrößerung umfasst, mit einem Beobachtungsstrahlengang, der die Mikroskop-Abbildungsoptik durchsetzt, wobei die Mikroskop-Abbildungsoptik einen konvergenten Beobachtungsstrahlengang aus dem Objektbereich in einen parallelen Strahlengang überführt, bei dem ein OCT-System zur Untersuchung des Objektbereichs vorgesehen ist.

Ein Operationsmikroskop der eingangs genannten Art ist aus der EP 0 815 801 B1 bekannt. Dort ist ein Operationsmikroskop mit einem von einem stereoskopischen Beobachtungsstrahlengang durchsetzten Mikroskop-Hauptobjektiv beschrieben, dem ein Zoomsystem für variable Vergrößerung zugeordnet ist. Das Operationsmikroskop enthält ein OCT-System. Dieses OCT-System umfasst eine Baugruppe zum Erzeugen eines OCT-Abtaststrahlengangs aus kurzkohärenter Laserstrahlung mit einer Analyseeinheit zur Auswertung von Interferenzsignalen. Dieser Baugruppe ist eine Einrichtung zum Scannen des OCT-Abtaststrahlengangs zugeordnet. Um mittels des OCT-Abtaststrahlengangs einen Operationsbereich abzutasten, enthält die Einrichtung zum Scannen zwei Scanspiegel, die um zwei Bewegungsachsen verstellt werden können. Bei dem Operationsmikroskop nach der EP 0 815 801 B1 wird der OCT-Abtaststrahlengang über einen Teilerspiegel in den Beleuchtungsstrahlengang des Operationsmikroskops eingekoppelt und mit diesem durch das Mikroskop-Hauptobjektiv hindurch zum Objektbereich gelenkt.

Dieses Operationsmikroskop bildet für die Erfindung den nächsten Stand der Technik.

Eine nichtinvasive Untersuchung und Messung von Strukturen innerhalb eines biologischen Gewebes ermöglicht die Methode der optischen Kohärenztomographie. Als optisches bildgebendes Verfahren ermöglicht die optische Kohärenztomographie mit einem entsprechenden OCT-System insbesondere das Generieren von Schnitt- oder Volumenbildern biologischen Gewebes mit Mikrometerauflösung. Ein entsprechendes OCT-System umfasst eine Quelle für zeitlich inkohärentes und räumlich kohärentes Licht mit einer Kohärenzlänge $1_c$, die einem Probenstrahlengang und einem Referenzstrahlengang zugeführt wird. Der Probenstrahlengang ist auf das zu untersuchende Gewebe gerichtet. Laserstrahlung, die aufgrund von Streuzentren im Gewebe in den Probenstrahlengang zurückgestrahlt wird, überlagert das OCT-System mit Laserstrahlung aus dem Referenzstrahlengang. Durch die Überlagerung entsteht ein Interferenzsignal. Aus diesem Interferenzsignal lässt sich die Position von Streuzentren für die Laserstrahlung im untersuchten Gewebe bestimmen.

Für OCT-Systeme ist das Bauprinzip des "Time-Domain OCT" und des "Fourier-Domain OCT" bekannt.

Der Aufbau eines "Time-Domain OCT" ist beispielsweise in der US 5,321,501 anhand von Fig. 1a auf Sp. 5, Z. 40 - Sp. 11, Z. 10 beschrieben. In einem solchen System wird die optische Weglänge des Referenzstrahlenganges über einen schnell beweglichen Referenzspiegel fortlaufend variiert. Das Licht aus Proben- und Referenzstrahlengang wird auf einem Photodetektor überlagert. Wenn die optischen Weglängen von Proben- und Referenzstrahlengang übereinstimmen, entsteht auf dem Photodetektor ein Interferenzsignal.

Ein "Fourier-Domain OCT" ist beispielsweise in der WO 2006/10544 A1 erläutert. Um die optische Weglänge eines Probenstrahlenganges zu vermessen, wird wiederum Licht aus dem Probenstrahlengang Licht aus einem Referenzstrahlengang überlagert. Im Unterschied zu einem "Time-Domain OCT" wird jedoch für eine Messung der optischen Weglänge des Probenstrahlenganges das Licht aus Proben- und Referenzstrahlengang nicht direkt einem Detektor zugeführt, sondern zunächst mittels eines Spektrometers spektral zerlegt. Die so erzeugte spektrale Intensität des überlagerten Signals aus Proben- und Referenzstrahlengang wird dann mit einem Detektor erfasst. Durch Auswerten des Detektorsignals kann wiederum die optische Weglänge des Probenstrahlenganges ermittelt werden.

Die EP 1 220 004 B1 offenbart ein Operationsmikroskop, das einer Beobachtungsperson durch Einblick in ein Okular die Untersuchung eines Operationsbereichs mit stereoskopischem Beobachtungsstrahlengang ermöglicht. Das Operationsmikroskop enthält eine Einrichtung zur Dateneinspiegelung mit einem Display und einem als Teilerwürfel ausgebildeten Strahlenteiler. Dieser Strahlenteiler ist im Grundkörper des Operationsmikroskops im parallelen Beobachtungsstrahlengang zwischen dem Mikroskop-Hauptobjektiv und dem Okular angeordnet. Er überlagert ein mit einer Displayoptik nach unendlich abgebildetes Displaybild dem parallelen Beobachtungsstrahlengang im Operationsmikroskop.

Aufgabe der Erfindung ist es, ein kompakt aufgebautes Operationsmikroskop mit variabler Vergrößerung bereitzustellen, bei dem das Erfassen von Tiefenbildern eines Objektbereichs mit einem OCT-Abtaststrahlengang möglich ist, dessen Verlauf dem Verlauf des optischen Beobachtungsstrahlengangs im Operationsmikroskop entspricht, der für eine Beobachtungsperson in einem Okulareinblick ein vergrößertes Bild des Objektbereichs bewirkt, wobei sich der Querschnitt des OCT-Abtaststrahlengangs im Objektbereich an die gewählte Vergrößerung anpasst.

Diese Aufgabe wird durch ein Operationsmikroskop der eingangs genannten Art gelöst, bei dem das OCT-System einen OCT-Abtaststrahlengang bereitstellt, der Kennzeichengemäß durch die Mikroskop-Abbildungsoptik geführt ist.

Auf diese Weise wird insbesondere bei einem unebenen Objektbereich gewährleistet, dass der optische Beobachtungsstrahlengang und die OCT-Abtaststrahlung identische Zonen des Objektbereichs abdek-

ken. Mittels der OCT-Abtaststrahlung kann somit genau das Beobachtungsbild abgetastet werden, das sich einer Beobachtungsperson im Binokulareinblick eines entsprechenden Operationsmikroskops darstellt. Dabei können Tiefenschnitte des Objektbereichs erfasst werden, die auf OCT-Abtaststrahlung basieren.

In Weiterbildung der Erfindung ist ein Einkoppelelement vorgesehen, das den OCT-Abtaststrahlengang in den Beobachtungsstrahlengang einkoppelt, um den OCT-Abtaststrahlengang dem Beobachtungsstrahlengang überlagert durch die Mikroskop-Abbildungsoptik zum Objektbereich zu führen. Vorzugsweise ist das Einkoppelelement als Teilerspiegel, insbesondere als Planspiegel oder Teilerwürfel ausgebildet. Auf diese Weise wird ein Mitbeobachter stets eine freie Sicht zum Objektbereich ermöglicht.

In Weiterbildung ist zwischen der Mikroskop-Abbildungskoptik und dem Einkoppelelement ein Auskoppelelement angeordnet, um Bildinformation aus dem Beobachtungsstrahlengang auszukoppeln.

In Weiterbildung der Erfindung ist zwischen dem Einkoppelelement und dem Mikroskop-Hauptobjektiv ein afokales Linsensystem angeordnet, das vorzugsweise als Zoomsystem ausgebildet ist. Auf diese Weise ist es möglich, abgestimmt auf das Beobachtungsbild, welches sich einer Beobachtungsperson im Okulareinblick des Operationsmikroskops darstellt, die laterale Auflösung der mit dem OCT-System gewinnbaren OCT-Information zu variieren.

In Weiterbildung der Erfindung umfasst das OCT-System für das Scannen des OCT-Abtaststrahlengangs einen ersten Scanspiegel. Vorzugsweise ist zusätzlich ein zweiter Scanspiegel vorgesehen, wobei der erste Scanspiegel um eine erste Drehachse bewegt werden kann und der zweite Scanspiegel sich um eine zweite Drehachse bewegen lässt. Dabei stehen die erste und die zweite Drehachse seitlich versetzt zueinander unter einem rechten Winkel. Auf diese Weise ist ein Abscannen eines Objektbereichs entsprechend einem senkrecht verlaufenden Rastermuster möglich.

In Weiterbildung der Erfindung umfasst das OCT-System einen Lichtleiter, der einen Lichtaustrittsabschnitt für den OCT-Abtaststrahlengang aufweist, wobei Mittel zum Bewegen des Lichtaustrittsabschnitts des Lichtleiters vorgesehen sind. Auf diese Weise lässt sich eine OCT-Abtastebene im Objektbereich variieren und es ist möglich, das System für unterschiedliche OCT-Wellenlängen in anbetracht der für sichtbares Licht ausgelegten optischen Komponenten im Beobachtungsstrahlengang für Mitbeobachtung einzustellen.

In Weiterbildung der Erfindung ist in dem OCT-Abtaststrahlengang für das Einstellen einer geometrischen Abbildung eines Austrittsendes eines Lichtleiters in eine OCT-Abtastebene ein verstellbares optisches System vorgesehen. Auf diese Weise kann die OCT-Abtastebene des Operationsmikroskops relativ zur Beobachtungsebene der optischen Beobachtungsstrahlengänge des Systems verlagert werden und es ist möglich, bei Verstellen des Abbildungsmaßstabes für die Mikroskop-Abbildungsoptik den Abbildungsmaßstab für die OCT-Abtaststrahlung so nachzujustieren, dass der Abbildungsmaßstab im optischen Beobachtungsstrahlengang dem Abbildungsmaßstab im OCT-Abtaststrahlengang entspricht. Vorzugsweise können die betreffenden Abbildungsmaßstäbe so identisch gehalten werden.

In Weiterbildung der Erfindung ist dem verstellbaren optischen Element eine Antriebseinheit zugeordnet. Auf diese Weise kann beispielsweise die OCT-Abtastebene um einen vorgegebenen Betrag relativ zur Beobachtungsebene des Operationsmikroskops variiert werden.

In Weiterbildung der Erfindung ist das OCT-System für das Bereitstellen eines ersten OCT-Abtastlichtstrahls mit einer ersten Wellenlänge und für das Bereitstellen eines zweiten OCT-Abtastlichtstrahles mit einer von der ersten Wellenlänge verschiedenen zweiten Wellenlänge ausgelegt. Auf diese Weise kann das Operationsmikroskop für die Untersuchung unterschiedlicher Gewebestrukturen und Körperorgane eines Patienten optimiert werden.

In Weiterbildung der Erfindung sind ein erstes und ein zweites OCT-System vorgesehen, die OCT-Abtastlichtstrahlen unterschiedlicher Wellenlänge bereitstellen. Auf diese Weise ist eine Untersuchung eines Objektbereichs auf der Grundlage unterschiedlicher OCT-Wellenlängen mit maximaler Auflösung möglich. Insbesondere kann so Gewebe bei unterschiedlicher Eindringtiefe mit OCT-Abtastlichtstrahlen untersucht werden. Außerdem ist so möglich, das Operationsmikroskop für verschiedene Applikationen auszulegen.

In Weiterbildung der Erfindung ist eine Kopplung von Mikroskop-Abbildungsoptik und einem OCT-System vorgesehen, um bei einer Veränderung des Arbeitsabstandes des Operationsmikrokops eine entsprechende Änderung der optischen Weglänge im OCT-System einzustellen. Auf diese Weise wird gewährleistet, dass ein durch die Mikroskop-Abbildungsoptik scharf abgebildeter Objektbereich auch mit dem OCT-System abgetastet werden kann.

In Weiterbildung der Erfindung ist eine Kopplung von Mikroskop-Abbildungsoptik und Kollimationsoptik des OCT-Systems vorgesehen, um den Abbildungsmaßstab im optischen Beobachtungsstrahlengang und den Abbildungsmaßstab im OCT-Abtaststrahlengang aneinander anzupassen. Auf diese Weise wird gewährleistet, dass der OCT-Abtaststrahlengang den in den optischen Beobachtungsstrahlengängen sichtbaren Beobachtungsbereich abtastet.

Vorteilhafte Ausführungsformen der Erfindung sind in den Figuren dargestellt und werden nachfolgend beschrieben.

Es zeigen:

Fig. 1     ein erstes Operationsmikroskop mit integriertem OCT-System;

Fig. 2     einen Schnitt des Mikroskop-Hauptobjektivs im Operationsmikroskop entlang der Linie II - II aus Fig. 1;

Fig. 3     einen Abschnitt des Operationsmikroskops mit einem ersten und einem zweiten OCT-System;

Fig. 4     eine Intensitätsverteilung des aus dem Lichtleiter eines OCT-Systems im Operationsmikroskop austretenden OCT-Abtastlichtstrahls; und

Fig. 5     eine Intensitätsverteilung des OCT-Abtastlichtstrahls in der OCT-Abtastebene im Objektbereich des Operationsmikroskops.

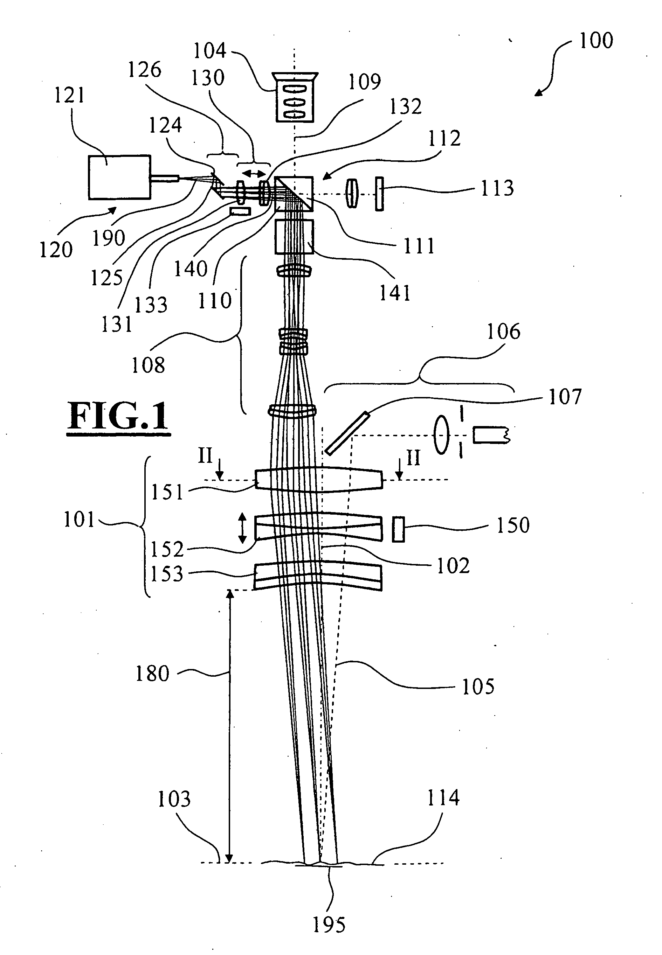

Das Operationsmikroskop 100 in Fig. 1 hat ein mittels Stellantrieb 150 fokussierbares Mikroskop-Hauptobjektivsystem 101 mit einer optischen Achse 102 sowie einer entsprechend verlagerbaren Fokusebene 103. Das Mikroskop-Hauptobjektivsystem 101 umfasst Teiloptiken 151, 152 und 153. Es wird von stereoskopischen Beobachtungsstrahlengängen eines Binokulartubus 104 und dem Beleuchtungsstrahlengang 105 einer Beleuchtungseinrichtung 106 mit einem Beleuchtungsspiegel 107 durchsetzt. Dieser Beleuchtungsspiegel ist auf der objektabgewandten Seite des Mikroskop-Hauptobjektivsystems 101 angeordnet.

Dem Binokulartubus 104 ist ein zoombares Vergrößerungssystem 108 zugeordnet. Das Mikroskop-Hauptobjektivsystem 101 und das zoombare Vergrößerungssystem 108 bilden die Mikroskop-Abbildungsoptik des Operationsmikroskops 100. Die Fig. 1 zeigt einen rechten Beobachtungsstrahlengang 109 des stereoskopischen Beobachtungsstrahlengangs im Operationsmikroskop 100.

Zwischen dem Binokulartubus 104 und dem zoombaren Vergrößerungssystem 108 befindet sich im parallelen, rechten Beobachtungsstrahlengang 109 ein erster Teilerwürfel 112, der aus rechtwinkligen Prismen 110, 111 aufgebaut ist. Ein mit dem ersten Teilerwürfel 112 identischer Teilerwürfel ist an entsprechender Position im parallelen, linken Beobachtungsstrahlengang angeordnet. Der erste Teilerwürfel 112 und der zweite Teilerwürfel haben eine Doppelfunktion: Sie wirken als Einkoppelelemente, das die Anzeige eines ersten Displays 113 und eines in Fig. 1 nicht gezeigten zweiten Displays in den rechten Beobachtungsstrahlengang 109 und den linken Beobachtungsstrahlengang des Operationsmikroskops einkoppelt.

Dem Bild des Objektbereichs 114 im Binokulartubus 104 wird somit die Anzeige des ersten Displays 113 und des zweiten Displays überlagert. Gleichzeitig wirken der erste Teilerwürfel 112 und der zweite Teilerwürfel als Elemente zur Einkopplung eines von einem ersten OCT-System 120 bereitgestellten OCT-Abtaststrahlengangs 190.

Das Operationsmikroskop 100 enthält ein erstes OCT-System 120 zur Aufnahme von OCT-Bildern. Das OCT-System umfasst eine Einheit 121 für die Erzeugung und Analyse eines OCT-Abtaststrahlengangs 190, der aus einem Lichtleiter 122 austritt. Der aus dem Lichtleiter 122 austretende Abtaststrahlengang 190 wird auf einen ersten Scanspiegel 124 und einen zweiten Scanspiegel 125 einer OCT-Scaneinheit 126 geführt. Er durchtritt nach der OCT-Scaneinheit 126 eine einstellbare Kollimationsoptik 130 mit einer Linse 131 und einer Linse 132. Die Kollimationsoptik 130 hat einen Antrieb 133 und bündelt den Abtaststrahlengang 190 zu einem parallelen Strahlenbündel 140.

Natürlich ist es auch möglich, mit dem ersten Scanspiegel 124 und dem zweiten Scanspiegel 125 der OCT-Scaneinheit 126 einen parallelen OCT-Abtaststrahlengang abzulenken. Hierfür bedarf es einer geeigneten Kollimationsoptik, etwa einer Sammellinse, die zwischen Lichtleiter 122 und OCT-Scaneinheit 126 angeordnet wird. Eine Kollimationsoptik 130, die sich auf der dem Lichtleiter abgewandten Seite der OCT-Scaneinheit 126 befindet, ist dann nicht erforderlich.

Das Strahlenbündel 140 aus der OCT-Scaneinheit 126 wird zu dem Teilerwürfel 112 im Beobachtungsstrahlengang 106 geführt. Der Teilerwürfel 112 ist im wesentlichen durchlässig für den für den Menschen sichtbaren Spektralbereich von Beobachtungslicht in diesem Beobachtungsstrahlengang. Er reflektiert jedoch den OCT-Abtaststrahlengang und überlagert diesen dem Beobachtungsstrahlengang 106. Es sei bemerkt, dass der Teilerwürfel 112 auch als Spiegelelement mit Planplatte ausgeführt werden könnte.

Das Licht des OCT-Abtaststrahlengangs 190 wird durch das Mikroskop-Hauptobjektiv 101 in einer OCT-Abtastebene 195 gebündelt. Die OCT-Abtastebene 195 ist die Ebene der durch die optischen Elemente im OCT-Abtaststrahlengang mit OCT-Scaneinheit 126, Sammellinse 130, Teilerwürfel 112 und Mikroskop-Hauptobjektiv 101 festgelegten geometrischen Abbildung des Austrittsendes von Lichtleiter 122 in den Objektbereich. Das heißt, das entsprechende geometrische Bild des Lichtleiter-Austrittsendes liegt in der OCT-Abtastebene 195.

Das in den OCT-Abtaststrahlengang zurückgestreute Licht gelangt über das Mikroskop-Hauptobjektiv 101, das zoombare Vergrößerungssystem 108 und den Teilerwürfel 112 zurück in die Einheit 121. Dort wird das aus dem Objektbereich 114 zurückgestreute OCT-Abtastlicht mit OCT-Strahlung aus einem Referenzstrahlengang interferiert. Das Interferenzsignal wird mittels eines Detektors erfasst und durch eine Rechnereinheit ausgewertet, die aus diesem Signal eine optische Weglängendifferenz zwischen Streuzentren für OCT-Licht im Objektbereich 114 und der Weglänge von Licht im Referenzzweig bestimmt.

Bei einer Variation des Arbeitsabstands 180 von Operationsmikroskop 100 durch Verstellen des Mikroskop-Hauptobjektivsystems 101 wird auch die optische Weglänge der Abtaststrahlengänge von den betreffenden OCT-Systemen im Operationsmikroskop verändert. Der Stelltrieb 150 des fokussierbaren Mikroskop-Hauptobjektivsystems 101 ist deshalb mit den OCT-Systemen im Operationsmikroskop über eine Signalleitung elektrisch verbunden. Dies bewirkt eine Kopplung der OCT-Systeme mit dem Mikroskop-Hauptobjektivsystem um so bei Bedarf im Falle einer Variation des Operationsmikroskop-Arbeitsabstands 180 von Objektbereich 114 die optische Weglänge der Referenzstrahlengänge in den OCT-Systemen entsprechend anzupassen.

Um Bildinformation aus dem Objektbereich 114 einer Dokumentationseinheit 140 zuführen zu können, befindet sich zwischen dem zoombaren Vergrößerungssystem 108 und dem Teilerwürfel 112 ein Auskoppelelement 141.

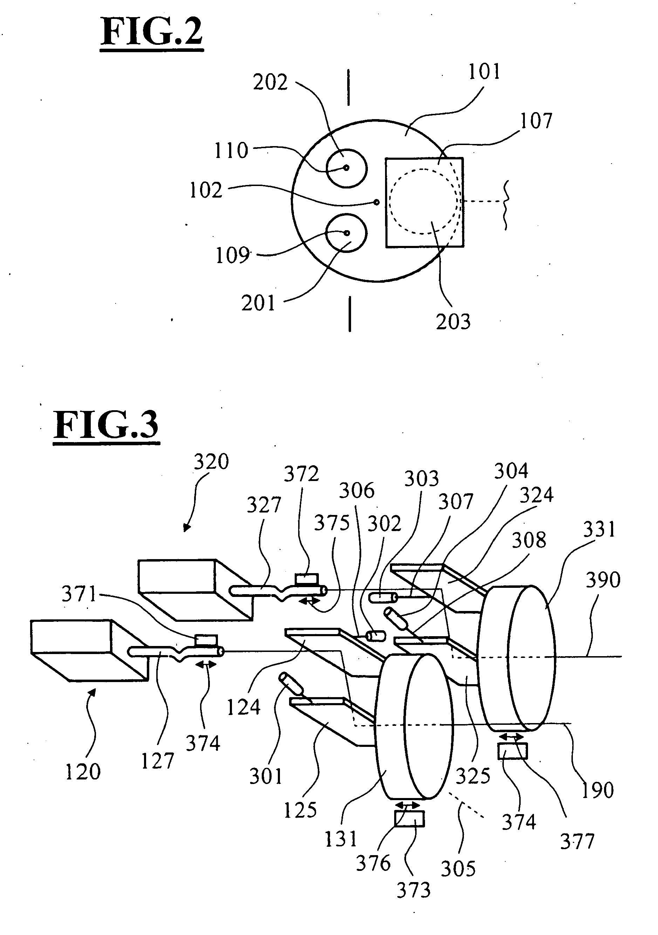

Fig. 2 ist ein Schnitt entlang der Linie II - II aus Fig. 1. Diese Figur erläutert den Verlauf der stereoskopischen Beobachtungsstrahlengänge im Operationsmikroskop 100 auf Fig. 1. Die optische Achse 102 des Mikroskop-Hauptobjektivs 101 liegt in dessen Zentrum. Der rechte Beobachtungsstrahlengang 109 und der linke Beobachtungsstrahlengang 110 durchsetzen das Mikroskop-Hauptobjektiv 101 mit dem vom Beleuchtungsspiegel 107 umgelenkten Beleuchtungsstrahlengang 105 in voneinander getrennten Schnittbereichen 201, 202 und 203.

Fig. 3 zeigt das erste OCT-System 120 und das zweite OCT-System 320 im Operationsmikroskop 100 aus Fig. 1. Wie das erste OCT-System 120 umfasst das zweite OCT-System 320 eine Einheit 321 für die Erzeugung und Analyse eines OCT-Abtaststrahlengangs. Der Wellenlängenbereich der OCT-Abtaststrahlengänge der beiden OCT-Systeme 120, 320 ist allerdings unterschiedlich: Das erste OCT-System basiert auf OCT-Abtaststrahlung der Wellenlänge $\lambda_1$ = 1310 *nm.* Das zweite OCT-System 320 arbeitet mit OCT-Abtaststrahlung der Wellenlänge $\lambda_2$ = 800 *nm.* Selbstverständlich könnten die OCT-Systeme auch für andere Arbeitswellenlängen ausgelegt werden. Arbeitswellenlängen lassen sich insbesondere im Bereich 600nm < $\lambda$ < 1500nm realisieren und sind je nach Applikation vorteilhaft.

Der OCT-Abtaststrahlengang 190 des ersten OCT-Systems 120 wird über die Sammellinse 130 in den ersten Teilerwürfel in den rechten Beobachtungsstrahlengang des Operationsmikroskops 100 eingekoppelt. Der OCT-Abtaststrahlengang 390 des zweiten OCT-Systems 320 wird über eine zweite Sammellinse in den zweiten Teilerwürfel im linken Beobachtungsstrahlengang des Operationsmikroskops 100 überlagert.

Wie das OCT-System 120 enthält hierzu das OCT-System 320 eine OCT-Scaneinheit 326 mit Scanspiegeln 324, 325 und einer Sammellinse 330, die den OCT-Abtaststrahlengang 390 zu einem parallelen Strahlenbündel sammelt.

Der erste Scanspiegel 124, 324 und der zweite Scanspiegel 125, 325 der OCT-Systeme 120, 320 sind mittels Stellantrieben 301, 302, 303, 304 um zwei zueinander senkrecht verlaufende Achsen 305, 306, 307 und 308 drehbeweglich angeordnet. Dies ermöglicht, die OCT-Abtaststrahlengänge 190, 390 unabhängig voneinander über eine Ebene zu scannen.

Um einer Bedienperson die Einstellung der OCT-Abtastebenen im Bezug auf die Objektebene der optischen Beobachtungsstrahlengänge im Operationsmikroskop 100 aus Fig. 1 zu ermöglichen, ist eine Verstellbarkeit der Sammellinsen 131, 331 und des Austrittsendes 133, 333 der Lichtleiter 127, 327 vorgesehen. Hierzu sind den Sammellinsen 131, 331 und den Lichtleitern 127, 327 Antriebseinheiten 371, 372, 373 und 374 zugeordnet. Mittels dieser Antriebseinheiten können die Sammellinsen 131, 331 und die Lichtleiter 127, 327 entsprechend der Doppelpfeile 374, 375, 376 und 377 verlagert werden. Insbesondere kann hierdurch nicht nur die Lage der OCT-Abtastebenen variiert werden, sondern es lässt sich auch eine Vergrößerung bzw. Verkleinerung des Austrittsendes der Lichtleiter 127, 327 auf einen gewünschten Wert einstellen.

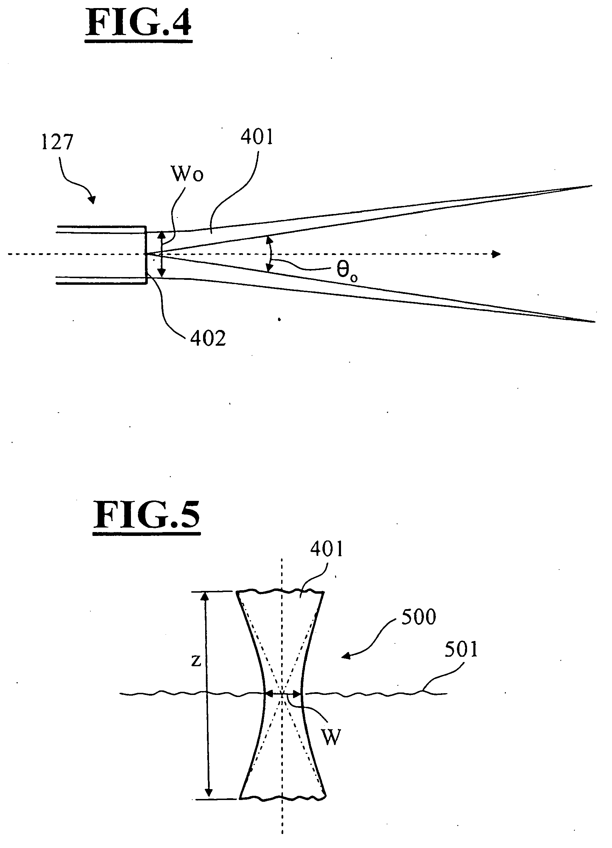

Fig. 4 zeigt den Frontabschnitt 402 des Lichtleiters 127 aus Fig. 1. Der Lichtleiter 127 wirkt für Licht der Wellenlänge $\lambda_1$ = 1310 *nm* als Monomodenfaser. Der Durchmesser d des Faserkerns des Lichtleiters 127 genügt der Beziehung

$$\frac{d}{2} < 2.4\,\frac{\lambda_1}{2\pi NA},$$

wobei NA die numerische Apertur der Frontfläche des Lichtleiters ist. Vorzugsweise liegt der Durchmesser d des Faserkerns des Lichtleiters 127 liegt deshalb im Bereich 5 $\mu$m < d < 10$\mu$m. In diesem Parameterbereich führt der Lichtleiter 127 das Licht mit gaussförmigen Wellenmoden. Aus dem Lichtleiter 127 tritt der OCT-Abtastlichtstrahl 401 mit einem näherungsweise gaussförmigen Strahlungsprofil aus, das durch einen Taillenparameter $W_1$ und einem Öffnungsparameter $\theta_1$ charakterisiert ist, wobei gilt:

$$\theta_1 = \frac{\lambda}{\pi W_1}$$

Für einen Faserkerndurchmesser von $d_1$ = 10 $\mu$m, einen Wellenlänge $\lambda_1$ = 1310nm ergibt sich damit als Maß für die Strahldivergenz ein Öffnungswinkel von $\theta_0 \approx$ 0,0827 rad.

Die Frontfläche 402 des Lichtleiters 122 wird über die Scanspiegel 124 und 125 im Operationsmikroskop 100 aus Fig. 1, die Sammellinse 130, den Teilerspiegel 150, den Umlenkspiegel 107 und das Mikroskop-Hauptobjektivsystem 101 auf den Objektbereich 108 in die OCT-Abtastebene 195 abgebildet.

Die Fig. 5 zeigt den Verlauf der Intensitätsverteilung des OCT-Abtastlichtstrahls 401 senkrecht zur OCT-Abtastebene 501. In der OCT-Abtastebene 501 hat die Intensitätsverteilung der OCT-Abtaststrahlung eine kleinste Einschnürung. Außerhalb der OCT-Abtastebene nimmt der Durchmesser des OCT-Abtaststrahlengangs zu. Da der OCT-Abtastlichtstrahl 401 aus dem Lichtleiter 122 aus Fig. 4 mit einem näherungsweise gaussförmigen Strahlungsprofil austritt, bewirken die Sammellinse 130 und das Mikroskop-Hauptobjektivsystem 101 für den OCT-Abtastlichtstrahl im Bereich der OCT-Abtastebene 160 ein sogenanntes Gaussbündel 500 des OCT-Abtastlichtstrahls 401. Dieses Gaussbündel 500 ist durch den konfokalen Parameter z als Maß für die longitudinale Ausdehnung der Taille des Gaussbündels, sowie den Taillenparameter W als Mass für den Durchmesser der kleinsten Einschnürung 502 des OCT-Abtastlichtstrahls 401, d.h. für den Durchmesser von dessen Taille charakterisiert, wobei gilt:

$$z = 2\frac{W^2\pi}{\lambda_1},$$

dabei ist $\lambda_1$ die Wellenlänge des OCT-Abtastlichtstrahls. Zwischen dem Taillenparametern W des Gaussbündels 500 und dem Taillenparameter $W_1$ des in Fig. 4 gezeigten, aus dem Lichtleiter 122 austretenden Abtastlichtstrahls 401 gilt die folgende Beziehung:

$$W = \beta W_1,$$

wobei $\beta$ der Vergrößerungs- bzw. Verkleinerungsparameter der oben erwähnten geometrischen Abbildung des Austrittsendes von Lichtleiter 122 aus Fig. 1 in der OCT-Abtastebene ist. $\beta$ ist mit der Brennweite $f_1$ der Kollimationsoptik 130 aus Fig. 1 und der Brennweite $f_2$ der Mikroskop-Abbildungsoptik mit Mikroskop-Hauptobjektivsystem 101 und Vergrößerungssystem 108 über die folgende Beziehung verknüpft:

$$\frac{f_2}{f_1} = \beta$$

Die verstellbare Kollimationsoptik der OCT-Systeme ermöglicht es, einer Änderung des Abbildungsmaßstabes der Mikroskop-Abbildungsoptik Abbildungsparameter $\beta$ für die entsprechenden OCT-Strahlengänge daran anzupassen. Vorzugsweise werden zur Anpassung hierfür die betreffenden Abbildungsmaßstäbe gleich gewählt.

Für ein bestimmtes Scann-Muster des OCT-Abtaststrahlengangs bewirkt dies, dass dieses dann automatisch an das Beobachtungsbild, das sich einer Beobachtungsperson im Okulareinblick des Operationsmikroskops 100 aus Fig. 1 bietet, anpasst.

Die Größe von Strukturen, die sich mit dem OCT-Abtastlichtstrahl 401 auflösen lassen, ist durch dessen Durchmesser in der OCT-Abtastebene 160, d.h. durch den Taillenparameter W bestimmt. Erfordert beispielsweise eine Applikation eine Lateralauflösung des OCT-Systems im Operationsmikroskop von ca. 40$\mu$m, muss nach dem Nyquist-Theoren der Querschnitt des OCT-Abtastlichtstrahls 401 auf der Oberfläche ca. 20$\mu$m betragen. Bei gegebener Wellenlänge $\lambda$ für den OCT-Abtastlichtstrahl 123 aus Fig. 1 müssen daher für eine gewünschte Auflösung des OCT-Systems 120 die Vergrößerung der optischen Abbildung im OCT-Strahlengang und der Durchmesser des Faserkerns im Lichtleiter 122 geeignet gewählt werden.

Der konfokale Parameter z als Maß für die longitudinale Ausdehnung der Taille des Gaussbündels bestimmt den axialen Tiefenbereich, aus dem in dem OCT-Abtaststrahlengang 123 aus Fig. 1 zurückgestreutes Licht detektiert werden kann: Je kleiner der konfokale Parameter z, desto größer ist der Verlust des OCT-Systems an lateraler Auflösung bei Entfernung eines mit OCT-Abtaststrahlung abgetasteten Objekts von der OCT-Abtastebene 160, da sich der Ort von Streuzentren nur innerhalb des durch den Taillenparamter W und den konfokalen Parameter z definierten "Trichter" lokalisieren lässt.

Nachdem die axiale Auflösung eines OCT-Systems einerseits durch die Kohärenzlänge $1_c$ des Lichts der im OCT-system eingesetzten Lichtquelle begrenzt ist, andererseits die laterale Auflösung des OCT-Systems abnimmt, wenn dessen Tiefenhub den konfokalen Parameter übersteigt, ist das Einstellen des konfokalen Parameters z auf den Tiefenhub des OCT-Systems günstig.

Für eine bestimmte Wellenlänge $\lambda$ des OCT-Abtastlichtstrahls 401 ergibt sich dann die mögliche laterale Auflösung des OCT-Systems aus Fig. 1, da die Wellenlänge $\lambda$ und der Rayleighparameter z den Taillenparameter W festlegen. Die Optikeinheiten im OCT-Abtaststrahlengang 123 aus Fig. 1 und die Bemaßung des Faserkerns von Lichtleiter 122 sind dann so zu wählen, dass sich der betreffende Taillenparamter ergibt. Entsprechendes gilt für den die Optikeinheiten im OCT-Abtaststrahlengang des zweiten OCT-Systems 320 in dem Operationsmikroskop.

Das Operationsmikroskop 100 aus Fig. 1 ist so ausgelegt, dass die Fokusebene 103 Mikroskop-Hauptobjektivs 101 für den sichtbaren Spektralbereich mit den OCT-Abtastebenen 195 der OCT-Systeme im Operationsmikroskop zusammenfällt. Dann liegt die in Fig. 5 gezeigte Taille 502 des betreffenden OCT-Abtastlichtstrahls in der Fokusebene des Operationsmikroskops.

Alternativ zu dieser Auslegung des Operationsmikroskops kann auch ein Versatz von OCT-Abtastebene und Fokusebene des Operationsmikroskops vorgesehen sein. Vorzugsweise ist dieser Versatz nicht größer als der konfokale Parameter des OCT-Abtastlichtstrahls im Bereich der OCT-Abtastebene. Dies ermöglicht es beispielsweise einen unmittelbar unter der Fokusebene des Operationsmikroskops liegenden Objektbereich mittels OCT zu visualisieren. Es kann aber auch sinnvoll sein, für bestimmte Anwendungen einen definierten Versatz vorzusehen, der den konfokalen Parameter übersteigt, etwa um mit dem Operationsmikroskop die Vorderseite der Cornea eines Patientenauges untersuchen zu können und gleichzeitig mittels des OCT-Systems die Rückseite der Cornea des Patientenauges oder dessen Linse zu visualisieren.

Indem die OCT-Abtastebene um den konfokalen Parameter z weiter vom Mikroskop-Hauptobjektivsystem 101 aus Fig. 1 entfernt ist, lässt sich der Tiefenhub für das OCT-System im Objektbereich maximieren.

## Patentansprüche

1. Operationsmikroskop (100)

   - mit einer Mikroskop-Abbildungsoptik (101, 108), die ein Mikroskop-Hauptobjektivsystem (101) sowie ein Vergrößerungssystem (108) mit variabler Vergrößerung umfasst;
   - mit einem Beobachtungsstrahlengang (109) zur Untersuchung eines Objektbereichs, der die Mikroskop-Abbildungsoptik (101, 103) durchsetzt; wobei
   - die Mikroskop-Abbildungsoptik (101, 108) einen konvergenten Beobachtungsstrahlengang (109) aus dem Objektbereich (114) in einen parallelen Strahlengang überführt; und
   - mit einem OCT-System (120, 320) zur Untersuchung des Objektbereichs (114),

   **dadurch gekennzeichnet, dass**

   - das OCT-System (120, 320) einen OCT-Abtaststrahlengang (190, 390) bereitstellt, der durch das Mikroskop-Hauptobjektivsystem und das Vergrößerungssystem des Mikroskop-Abbildungsoptik (101, 108) geführt ist.

2. Operationsmikroskop nach Anspruch 1, **dadurch gekennzeichnet, dass** ein Einkoppelelement (112) vorgesehen ist, das den OCT-Abtaststrahlengang (190, 390) in den Beobachtungsstrahlengang (109, 110) einkoppelt, um diesen dem Beobachtungsstrahlengang (109) überlagert durch die Mikroskop-Abbildungsoptik zum Objektbereich (114) zu führen.

3. Operationsmikroskop nach Anspruch 2, **dadurch gekennzeichnet, dass** eine Displayanzeige (113) vorgesehen ist und das Einkoppelelement (112) für den OCT-Abtaststrahlengang (190, 390) als Einkoppelelement für Displayinformation wirkt, um dem Beobachtungsstrahlengang (109) Displayinformation zu überlagern.

4. Operationsmikroskop nach Anspruch 2 oder 3, **dadurch gekennzeichnet, dass** das Einkoppelelement (112) als Teilerspiegel, insbesondere als Planspiegel oder Teilerwürfel ausgebildet ist.

5. Operationsmikroskop nach einem der Ansprüche 2 bis 4, **dadurch gekennzeichnet, dass** zwischen der Mikroskop-Abbildungsoptik (101, 108) und dem Einkoppelelement (112) ein Auskoppelelement (141) angeordnet ist, um Bildinformation aus dem Beobachtungsstrahlengang (109) auszukoppeln.

6. Operationsmikroskop nach einem der Ansprüche 1 bis 5, **dadurch gekennzeichnet, dass** das Vergrößerungssystem der Mikroskop-Abbildungsoptik als afokales Linsensystem (108) ausgebildet ist.

7. Operationsmikroskop nach Anspruch 6, **dadurch gekennzeichnet, dass** das afokale Linsensystem als Zoomsystem (108) ausgebildet ist.

8. Operationsmikroskop nach einem der Ansprüche 1 bis 7, **dadurch gekennzeichnet, dass** das OCT-System (120) für das Scannen des OCT-Abtaststrahlengangs (190) einen ersten Scanspiegel (124) umfasst, der um eine erste Drehachse (306) bewegt werden kann.

9. Operationsmikroskop nach Anspruch 8, **dadurch gekennzeichnet, dass** ein zweiter Scanspiegel (125) vorgesehen ist, der um eine zweite Drehachse (306) bewegt werden kann, und wobei die erste Drehachse (306) und die zweite Drehachse (305) seitlich versetzt in einem rechten Winkel zueinander stehen.

10. Operationsmikroskop nach einem der Ansprüche 1 bis 9, **dadurch gekennzeichnet, dass** das OCT-System einen Lichtleiter (127, 327) umfasst, der einen Lichtaustrittsabschnitt (402) für den OCT-Abtaststrahlengang (190, 390) aufweist, wobei Mittel (371, 372) zum Bewegen des Lichtaustrittsabschnitts (402) des Lichtleiters (127, 327) vorgesehen sind.

11. Operationsmikroskop nach einem der Ansprüche 1 bis 10, **dadurch gekennzeichnet, dass** das OCT-System eine Kollimationsoptik (130) umfasst, welche dem Einkoppelelement (112) einen parallelen Abtaststrahlengang (109) zuführt.

12. Operationsmikroskop nach Anspruch 11, **dadurch gekennzeichnet, dass** eine Kopplung von Mikroskop-Abbildungsoptik (101, 108) und Kollimationsoptik (130) des OCT-Systems (120) vorgesehen ist, um die Abbildungsmaßstäbe im Beobachtungsstrahlengaug (109) im OCT-Abtaststrahlengang (190) aneinander anzupassen.

13. Operationsmikroskop nach Anspruch 10, **dadurch gekennzeichnet, dass** in dem OCT-Abtaststrahlengang (190) für das Einstellen einer geometrischen Abbildung des Austrittsendes (402) des Lichtleiters (127, 327) in eine OCT-Abtastebene (195) ein verstellbares Optiksystem (130, 131, 331) vorgesehen ist.

14. Operationsmikroskop nach Anspruch 13, **dadurch gekennzeichnet, dass** dem verstellbaren Optiksystem (130, 131, 331) eine Antriebseinheit (373, 374) zugeordnet ist.

15. Operationsmikroskop nach einem der Ansprüche 1 bis 14, **dadurch gekennzeichnet, dass** das OCT-System für das Bereitstellen eines ersten OCT-Abtastlichtstrahls mit einer ersten Wellenlänge und für das Bereitstellen eines zweiten OCT-Abtastlichtstrahls mit einer von der ersten Wellenlängen verschiedenen zweiten Wellenlänge ausgelegt ist.

16. Operationsmikroskop nach einem der Ansprüche 1 bis 15, **dadurch gekennzeichnet, dass** ein erstes OCT-System (120) und ein zweites OCT-System (320) vorgesehen sind, die OCT-Abtastlichtstrahlen (190, 390) unterschiedlicher Wellenlänge bereitstellen.

17. Operationsmikroskop nach einem der Ansprüche 1 bis 16, **dadurch gekennzeichnet, dass** eine Kopplung von Mikroskop-Abbildungsoptik (101, 108) und OCT-System (120, 320) vorgesehen ist, um bei einer Veränderung des Arbeitsabstandes (180) des Ope-rationsmikroskops (100) eine entsprechende Ände-rung der optischen Weglänge des Referenzstrahlenganges im OCT-System (120, 320) einzustellen.

**Claims**

1. Operating microscope (100)

   - with an microscope imaging optical system (101, 108), which comprises a microscope main objective system (101) and a magnifying system (108) with variable magnification;
   - with an observation beam path (109) for examining an object region that passes through the microscope imaging optical system (101, 108);
   - the microscope imaging optical system (101, 108) converting a convergent observation beam path (109) from the object region (114) into a parallel beam path; and
   - with an OCT system (120, 320) for examining the object region (114),

**characterized in that**

   - the OCT system (120, 320) provides an OCT scanning beam path (190, 390) that is guided through the microscope main objective system and the magnifying system of the microscope imaging optical system (101, 108).

2. Operating microscope according to Claim 1, **characterized in that** a coupling-in element (112) is provided, which couples the OCT scanning beam path (190, 390) into the observation beam path (109, 110) in order to guide said OCT scanning beam path superposed on the observation beam path (109) through the microscope imaging optical system to the object region (114).

3. Operating microscope according to Claim 2, **characterized in that** a display (113) is provided and the coupling-in element (112) for the OCT scanning beam path (190, 390) acts as a coupling-in element for display information in order to superpose display information onto the observation beam path (109).

4. Operating microscope according to Claim 2 or 3, **characterized in that** the coupling-in element (112) is designed as a splitter mirror, more particularly as a plane mirror or splitter cube.

5. Operating microscope according to one of Claims 2 to 4, **characterized in that** a decoupling element (141) is arranged between the microscope imaging optical system (101, 108) and the coupling-in element (112) in order to decouple image information from the observation beam path (109).

6. Operating microscope according to one of Claims 1 to 5, **characterized in that** the magnifying system of the microscope imaging optical system is designed as an afocal lens system (108).

7. Operating microscope according to Claim 6, **characterized in that** the afocal lens system is designed as a zoom system (108).

8. Operating microscope according to one of Claims 1 to 7, **characterized in that** the OCT system (120) comprises a first scanning mirror (124) for scanning the OCT scanning beam path (190), which first scanning mirror can be moved about a first rotational axis (306).

9. Operating microscope according to Claim 8, **characterized in that** a second scanning mirror (125) that can be moved about a second rotational axis (305) is provided, and the first rotational axis (306) and the second rotational axis (305) being laterally offset and at a right angle with respect to one another.

10. Operating microscope according to one of Claims 1 to 9, **characterized in that** the OCT system comprises an optical waveguide (127, 327), which has a light emission section (402) for the OCT scanning beam path (190, 390), with means (371, 372) being provided for moving the light emission section (402) of the optical waveguide (127, 327).

11. Operating microscope according to one of Claims 1 to 10, **characterized in that** the OCT system comprises a collimation optical system (130), which supplies a parallel scanning beam path (109) to the coupling-in element (112).

12. Operating microscope according to Claim 11, **characterized in that** provision is made for coupling the microscope imaging optical system (101, 108) and the collimation optical system (130) of the OCT system (120) in order to match the imaging scales in the observation beam path (109) and in the OCT scanning beam path (190) to each other.

13. Operating microscope according to Claim 10, **characterized in that** an adjustable optical system (130, 131, 331) is provided in the OCT scanning beam path (190) for setting a geometric image of the emission end (402) of the optical waveguide (127, 327) in an OCT scanning plane (195).

14. Operating microscope according to Claim 13, **characterized in that** a drive unit (373, 374) is assigned to the adjustable optical system (130, 131, 331).

15. Operating microscope according to one of Claims 1 to 14, **characterized in that** the OCT system is designed for the provision of a first OCT scanning light beam at a first wavelength and for the provision of a second OCT scanning light beam at a second wavelength that differs from the first wavelength.

16. Operating microscope according to one of Claims 1 to 15, **characterized in that** provision is made for a first OCT system (120) and a second OCT system (320) that provide OCT scanning light beams (190, 390) with different wavelengths.

17. Operating microscope according to one of Claims 1 to 16, **characterized in that** provision is made for coupling the microscope imaging optical system (101, 108) and the OCT system (120, 320) in order to set a corresponding change in the optical path length of the reference beam path in the OCT system (120, 320) if the working distance (180) of the operating microscope (100) changes.

**Revendications**

1. Microscope d'opération (100)

   - avec une optique de reproduction du microscope (101, 108) comportant un système d'objectif principal du microscope (101) ainsi qu'un système de grandissement (108) à grandissement variable ;
   - avec un chemin optique d'observation (109) pour l'examen d'un espace objet effectué par l'optique de reproduction du microscope (101, 108) ;
   - l'optique de reproduction du microscope (101, 108) transformant un chemin optique d'observation convergent (109) à partir de l'espace objet (114) en un chemin optique parallèle ; et
   - avec un système OCT (120, 320) pour l'examen de l'espace objet (114),

   **caractérisé en ce que**

   - le système OCT (120, 320) fournit un chemin optique d'exploration OCT (190, 390) qui est acheminé à travers le système d'objectif principal du microscope et le système de grandissement de l'optique de reproduction du microscope (101, 108).

2. Microscope d'opération selon la revendication 1, **caractérisé en ce qu'**il est prévu un élément de couplage d'entrée (112) qui couple le chemin optique d'exploration OCT (190, 390) en entrée dans le chemin optique d'observation (109, 110) pour acheminer celui-ci, superposé au chemin optique d'observation (109), à travers l'optique de reproduction du microscope jusqu'à l'espace objet (114).

3. Microscope d'opération selon la revendication 2, **caractérisé en ce qu'**il est prévu un affichage (113) et que l'élément de couplage d'entrée (112) pour le chemin optique d'exploration OCT (190, 390) agit comme élément de couplage d'entrée pour les informations d'affichage pour superposer des informations d'affichage au chemin optique d'observation (109).

4. Microscope d'opération selon la revendication 2 ou 3, **caractérisé en ce que** l'élément de couplage d'entrée (112) est réalisé sous forme de miroir diviseur, notamment de miroir plan ou de cube diviseur.

5. Microscope d'opération selon une des revendications 2 à 4, **caractérisé en ce qu'**un élément de couplage vers l'extérieur (141) est disposé entre l'optique de reproduction du microscope (101, 108) et l'élément de couplage vers l'intérieur (112) pour coupler l'information d'image du chemin optique d'observation (109) vers l'extérieur.

6.  Microscope d'opération selon une des revendications 1 à 5, **caractérisé en ce que** le système de grandissement de l'optique de reproduction du microscope est réalisé sous la forme d'un système afocal de lentilles (108).

7.  Microscope d'opération selon la revendication 6, **caractérisé en ce que** le système afocal de lentilles est réalisé sous la forme d'un système zoom (108).

8.  Microscope d'opération selon une des revendications 1 à 7, **caractérisé en ce que** le système OCT (120) pour le balayage du chemin optique d'exploration OCT (190) comporte un premier miroir de balayage (124) mobile autour d'un premier axe de rotation (306).

9.  Microscope d'opération selon la revendication 8, **caractérisé en ce qu'**un deuxième miroir de balayage (125) mobile autour d'un deuxième axe de rotation (305) est prévu, le premier axe de rotation (306) et le deuxième axe de rotation (305) étant mutuellement perpendiculaires décalés latéralement.

10. Microscope d'opération selon une des revendications 1 à 9, **caractérisé en ce que** le système OCT comporte un conduit de lumière (127, 327) qui présente une section de sortie de lumière (402) pour le chemin optique d'exploration OCT (190, 390), des moyens (371, 372) étant prévus pour mouvoir la section de sortie de lumière (402) du conduit de lumière (127, 327).

11. Microscope d'opération selon une des revendications 1 à 10, **caractérisé en ce que** le système OCT comporte une optique de collimation (130) qui achemine un chemin optique d'exploration parallèle (109) vers l'élément de couplage d'entrée (112).

12. Microscope d'opération selon la revendication 11, **caractérisé en ce qu'**il est prévu un accouplement de l'optique de reproduction du microscope (101, 108) avec l'optique de collimation (130) du système OCT (120) pour adapter entre elles les échelles de reproduction dans le chemin optique d'observation (109) et dans le chemin optique d'exploration OCT (190).

13. Microscope d'opération selon la revendication 10, **caractérisé en ce qu'**un système optique réglable (130, 131, 331) est prévu dans le chemin optique d'exploration OCT (190) pour le réglage d'une reproduction géométrique de l'extrémité de sortie (402) du conduit de lumière (127, 327) dans un plan d'exploration OCT (195).

14. Microscope d'opération selon la revendication 13, **caractérisé en ce qu'**une unité d'actionnement (373, 374) est associée au système optique réglable (130, 131, 331).

15. Microscope d'opération selon une des revendications 1 à 14, **caractérisé en ce que** le système OCT est conçu pour la fourniture d'un premier rayon lumineux d'exploration OCT avec une première longueur d'ondes et pour la fourniture d'un deuxième rayon lumineux d'exploration OCT avec une deuxième longueur d'ondes différente de la première longueur d'ondes.

16. Microscope d'opération selon une des revendications 1 à 15, **caractérisé en ce qu'**il est prévu un premier système OCT (120) et un deuxième système OCT (320) pour la fourniture de rayons lumineux d'exploration OCT (190, 390) de différentes longueurs d'ondes.

17. Microscope d'opération selon une des revendications 1 à 16, **caractérisé en ce qu'**il est prévu un couplage de l'optique de reproduction du microscope (101, 108) avec le système OCT (120, 320) pour régler, lors d'une modification de la distance de travail (180) du microscope d'opération (100), une modification correspondante de la longueur du chemin optique du chemin optique de référence dans le système OCT (120, 320).

**FIG.1**

# FIG.2

# FIG.3

# FIG.4

127

401

Wo

402

$\theta_0$

# FIG.5

401

500

501

z

W

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0815801 B1
- US 5321501 A
- WO 200610544 A1
- EP 1220004 B1